# EUROPEAN PATENT APPLICATION

(11) **EP 4 834 230 A1**
(43) Date of publication of application: **04.11.2026**
(21) Application number: 24906578.0
(22) Date of filing: 20.12.2024
(51) Int. Cl.: A61B 6/03, A61B 6/58

(54) **SCATTERING CORRECTION METHOD AND SCATTERING CORRECTION METHOD SYSTEM FOR STATIC CT**

(30) Priority: 21.12.2023 CN 202311773367
(71) Applicant: Nanovision (Nanjing) Technology Group Co., Ltd., Nanjing, Jiangsu 211102 (CN)
(72) Inventor: LIANG, Song, Beijing 100094 (CN); CHEN, Geng, Beijing 100094 (CN); CUI, Zhili, Beijing 100094 (CN); LI, Yunxiang, Beijing 100094 (CN); QI, Yanjun, Beijing 100094 (CN)
(74) Representative: Wang, Bo
(86) International application number: PCT/CN2024/141244
(87) International publication number: WO 2025/131108

(57) **Abstract**

Disclosed are a scattering correction method and a scattering correction system for static CT. The scattering correction method comprises: acquiring scattering data in advance by means of a scattering detector arranged on at least one side of a main detector to obtain a scattering correction table, and obtaining a pre-training model by means of model training, wherein the scattering correction table comprises scattering distribution corresponding to each bed position and angle; obtaining an original image of a user; pre-correcting the original image to obtain a first image; eccentrically cutting the first image to obtain a second image; determining whether a CT scanning mode of the user is helical scanning or not; if the result is yes, carrying out scattering correction on the second image by using the scattering correction table; and if the result is no, obtaining, on the basis of the pre-training model, scattering distribution corresponding to the second image, and carrying out scattering correction on the second image. According to the scattering correction method, interpolation is used to obtain the scattering correction table, enabling precise scattering correction and improving the scattering correction effect.

## Description

### BACKGROUND

### Technical Field

The present invention belongs to the field of radiation imaging technologies, and relates to a scatter correction method for static CT and a corresponding scatter correction system for static CT.

### Related Art

Currently, a variety of methods have been developed for CT scatter correction, such as the beam stop array (BSA) method and the scatter kernel convolution (SKS) method. In the BSA method measures scatter by means of pre-designed two-dimensional grid beam stoppers, and obtains the scatter distribution via interpolation to implement scatter correction. This method requires two separate imaging scans, that is, one with the BSA and the other without the BSA, which prolongs the scanning time, increases additional dose, and may also increase errors caused by object motion. The SKS method requires no hardware adjustment or additional scanning and can achieve desirable scatter estimation. Nevertheless, the method involves complicated parameter tuning and suffers from instability, and has poor estimation accuracy for scatter from complex objects and cross scatter in multi-source scanning scenarios.

However, in view of the diversity and complexity of scatter suppression, there is currently no mature standard method well adapted to static CT. The static CT adopts a dual-ring structure. Since a plurality of radiation sources perform imaging on the same detector, ray angles change drastically, making the anti-scatter grid commonly used in conventional helical CT inapplicable for scatter suppression. Accordingly, it is difficult to design a proper anti-scatter grid for static CT to suppress scatter. Consequently, the detectors receive a large quantity of scatter rays. Especially under the multi-source exposure mode, severe cross scatter occurs and generates prominent scatter artifacts, which greatly degrade image quality.

### SUMMARY

A primary technical problem to be resolved in the present invention is to provide a scatter correction method for static CT.

Another technical problem to be resolved in the present invention is to provide a scatter correction system for static CT.

To achieve the foregoing technical objectives, the present invention adopts the following technical solutions.

According to a first aspect of embodiments of the present invention, a scatter correction method for static CT is provided, including the following steps:
collecting, by a scatter detector arranged on at least one side of a main detector, scatter data in advance, to obtain a scatter correction table through interpolation, and performing model training through the scatter data, to obtain a pre-trained model, where the scatter correction table includes a scatter distribution corresponding to each bed position and each angle;
obtaining an original image of a user for performing a static CT scan;
pre-correcting the original image to obtain a first image;
eccentrically cropping the first image to obtain a second image;
determining whether a CT scanning mode of the user is helical scanning;
performing scatter correction on the second image by using the scatter correction table if a determining result is yes; and obtaining a scatter distribution corresponding to the second image based on the pre-trained model, and performing scatter correction on the second image, if a determining result is no.

Preferably, the scatter correction table is obtained in the following manner:
collecting a scatter image based on the scatter detector arranged on the at least one side of the main detector, and performing data preprocessing;
extracting, into an array, scatter data based on preprocessed data, and causing the scatter data to correspond to the bed position and the angle, where the array includes data information of each bed position and each angle;
extracting, based on the scatter data, all scatter values of each pixel, and a bed position and an angle corresponding to each pixel;
obtaining, within a bed position and angle plane, a scatter value of each grid position within the plane through interpolation based on a set bed position and angle grid;
arranging, based on a detection direction of the main detector, the scatter values obtained through interpolation, and performing smoothing processing; and
storing scatter distributions and corresponding bed positions and the angles of all the main detectors in the detection direction, to form the scatter correction table.

Preferably, the performing scatter correction based on the scatter correction table specifically includes:
inputting the second image;
extracting bed position information and angle information of the second image;
converting a detection angle into a range of 0 degrees to 360 degrees for each row of main detectors, and performing calculation based on the bed position and a quantity of rows of the second image, to obtain bed positions corresponding to the row of main detectors;
finding, in the scatter correction table based on the bed position and the angle information corresponding to the row of detectors, scatter values corresponding to each row of detectors, to obtain the scatter distribution corresponding to the second image;
performing anti-logarithmic transform on the second image, and performing reciprocal calculation, to obtain an image without being subjected to scatter correction;
subtracting, from the obtained image without being subjected to the scatter correction, the scatter distribution corresponding to the second image, to obtain a scatter-corrected image; and
performing a negative logarithm operation on the scatter-corrected image, to complete the scatter correction on the second image.

Preferably, the obtaining, based on the pre-trained model, the scatter distribution corresponding to the second image specifically includes:
obtaining the scatter data collected by the scatter detector, and projection data corresponding to the second image;
inputting the scatter data and the projection image data together into the pre-trained model; and
outputting, based on the pre-trained model, the scatter distribution corresponding to the second image.

Preferably, the manner of interpolation includes at least cube interpolation or biharmonic spline interpolation, and compute unified device architecture (CUDA) acceleration is performed during the interpolation.

Preferably, the pre-correcting the original image specifically includes:
performing background correction on the original image, to obtain a background-corrected image;
performing gain correction on the background-corrected image, to obtain a gain-corrected image; and
performing air correction on the gain-corrected image, to obtain the first image.

Preferably, in an axial scanning mode, if both sides of the main detector are provided with scatter detectors, a scatter value of each pixel on the main detector is obtained through planar interpolation by using scatter values measured by the scatter detectors on both sides and a low-frequency characteristic of the scatter distribution, to perform scatter subtraction; or
if only one side of the main detector is provided with the scatter detector, a scatter value of each pixel on the main detector is estimated by using a scatter kernel convolution method or an AI method, to perform scatter subtraction.

Preferably, an arrangement spacing of the scatter detectors is greater than an arrangement spacing of the main detectors; and a larger arrangement spacing leads to a sparser arrangement, and a smaller arrangement spacing leads to a denser arrangement.

Preferably, the collecting, by a scatter detector, scatter data includes at least:
scatter data collected under exposure of a single radiation source; or scatter data collected under alternating exposure of a plurality of radiation sources; or scatter data collected under simultaneous exposure of a plurality of radiation sources.

According to a second aspect of embodiments of the present invention, a scatter correction system for static CT is provided, including a processor and a memory, where the processor reads a computer program in the memory, and is configured to perform the following operations:
collecting, by a scatter detector arranged on at least one side of a main detector, scatter data in advance, to obtain a scatter correction table through interpolation, and performing model training through the scatter data, to obtain a pre-trained model, where the scatter correction table includes a scatter distribution corresponding to each bed position and each angle;
obtaining an original image of a user for performing a static CT scan;
pre-correcting the original image to obtain a first image;
eccentrically cropping the first image to obtain a second image;
determining whether a CT scanning mode of the user is helical scanning;
performing scatter correction on the second image by using the scatter correction table if a determining result is yes; and obtaining a scatter distribution corresponding to the second image based on the pre-trained model, and performing scatter correction on the second image, if a determining result is no.

Compared with the prior art, the present invention has the following beneficial effects:
1. Scatter detectors are used to measure scatter in real time during CT scanning. By taking advantage of the characteristics of static CT, a scatter distribution at all positions and angles of a scanned object can be accurately obtained through interpolation, to perform precise scatter correction, thereby improving the effect of scatter correction.
2. The scatter correction method is easy to implement, and has low algorithm complexity.
3. The method may be widely applied to single-source and multi-source exposure modes.

### BRIEF DESCRIPTION OF THE DRAWINGS

FIG. 1A is a diagram of a principle of scatter detection according to an embodiment of the present invention;
FIG. 1B is a schematic diagram of an arrangement of a main detector and a scatter detector according to an embodiment of the present invention;
FIG. 1C is a schematic diagram of another arrangement of a main detector and a scatter detector according to an embodiment of the present invention;
FIG. 1D is a schematic diagram of still another arrangement of a main detector and a scatter detector according to an embodiment of the present invention;
FIG. 2 is a flowchart of obtaining a scatter correction table according to an embodiment of the present invention;
FIG. 3 is a schematic diagram of an entire curved surface obtained through interpolation based on a measured value according to an embodiment of the present invention;
FIG. 4 is a flowchart of a scatter correction method for static CT according to an embodiment of the present invention;
FIG. 5 is a flowchart of performing scatter correction based on a scatter correction table according to an embodiment of the present invention; and
FIG. 6 is a structure diagram of a scatter correction system for static CT according to an embodiment of the present invention.

### DETAILED DESCRIPTION

Technical content of the present invention is described in detail below with reference to the accompanying drawings and specific embodiments.

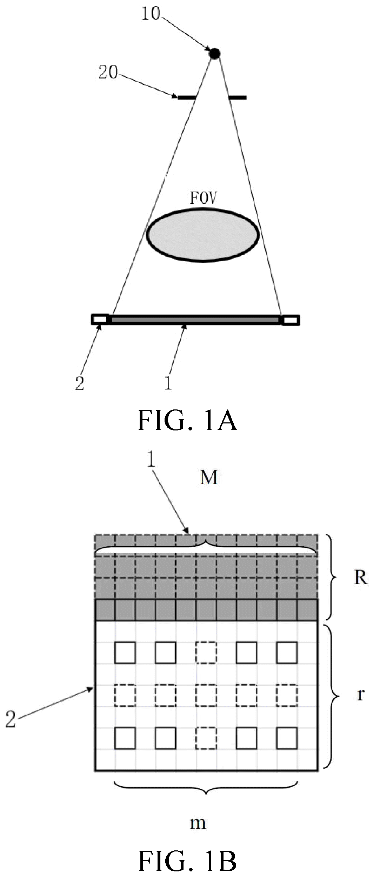

In embodiments of the present invention, a scatter detector is arranged on a main detector to measure a scatter component of a projection image. As shown in FIG. 1A, the detector is divided into a main detector 1 and scatter detectors 2. The main detector 1 is located in a middle portion of the detector, and the scatter detectors 2 are located on two sides of the main detector 1. In actual application, an emitted beam from an X-ray source 10 is constrained by a beam limiting device 20 to a predefined field of view (FOV). In this case, the scatter detector 2 is located outside the FOV and not covered by primary rays, and a signal measured by the scatter detector is from X-ray scatter signals generated after X-rays pass through an object under test.

It may be understood that, in this embodiment, the scatter detector 2 and the main detector 1 do not depend on a specific implementation form. For example, the scatter detector 2 may be an independently controlled detection unit separated from the main detector 1, or may be an independent photodiode (PD) array; or may be a detector pixel array (including but not limited to a PD+DMS, a CMOS+PD, a TFT PD, IGZO, a photon flux detector, CZT, a photon counting detector, and the like). In addition, the scatter detector 2 may also be arranged on only one side of the main detector 1.

In this embodiment, a pixel array of the main detector 1 is uniformly arranged. The pixel array of the main detector 1 is represented as I (R*M), that is, R rows and M columns. The arrangement of the scatter detectors 2 in a Z-X plane may be in a form of a sparse matrix, and a scatter matrix is represented as S (r*m), that is, r rows and m columns. When m=M, the main detector 1 and the scatter detector 2 have a same quantity of columns.

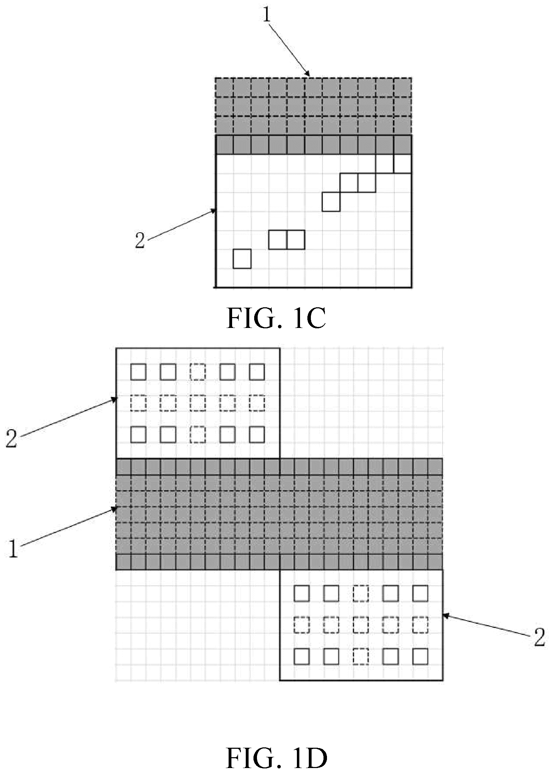

As shown in FIG. 1B to FIG. 1D, a part of one detector unit is used as an example for description. In FIG. 1B, scatter detectors 2 are arranged on only one side of a main detector 1, and pixel arrays of two scatter detectors 2 are sparse and symmetrically distributed. In FIG. 1C, one scatter detector 2 is arranged on only one side of a main detector 1, and a pixel array of the scatter detector 2 is sparse and asymmetrically distributed. In FIG. 1D, a scatter detector 2 is arranged on two sides of a main detector 1, pixel arrays of two scatter detectors 2 are sparse and symmetrically distributed, and the pixel arrays of two scatter detectors 2 on both sides are symmetrical about the center of the main detector 1.

It may be understood that, FIG. 1B to FIG. 1D merely show a partial arrangement of the main detectors 1 and the scatter detectors 2. In another embodiment, the arrangement of the main detectors 1 and the scatter detectors 2 may be adaptively adjusted as required, as long as scatter data can be collected for scatter correction.

When CT scanning is performed through static CT, the scatter data may be collected in real time by using the scatter detector 2, so as to collect the scatter data in advance by using an arranged scatter detector, obtain a scatter correction table through interpolation, and perform model training by using the scatter data, to obtain a pre-trained model. Further, in actual application, scatter correction is performed on an original image of a patient based on the scatter correction table and the pre-trained model, to obtain a high-quality to-be-detected image.

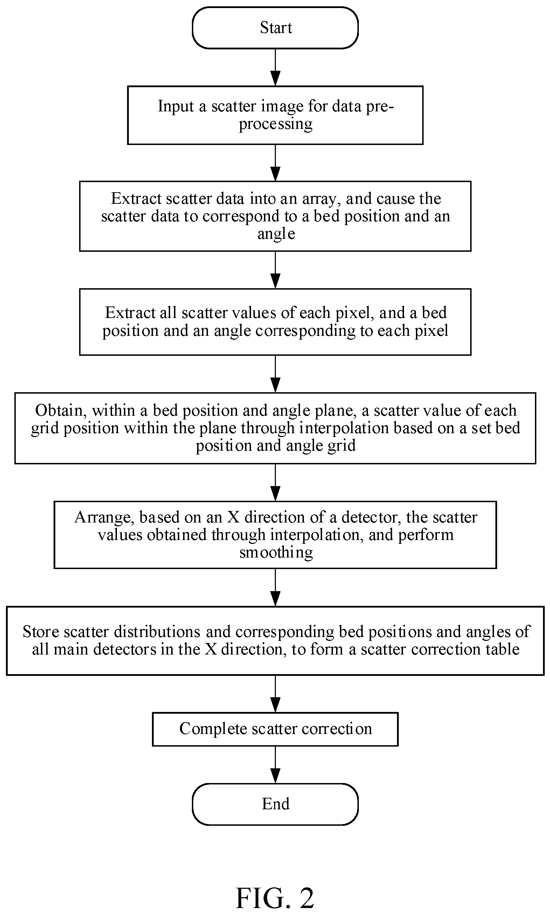

Referring to FIG. 2, in an embodiment of the present invention, a scatter correction table is obtained through steps S10 to S60.

First, a scatter image is collected based on a scatter detector arranged on at least one side of a main detector, and data preprocessing is performed.

The scatter data collected by the scatter detector includes at least: scatter data collected under exposure of a single radiation source; or scatter data collected under alternating exposure of a plurality of radiation sources; or scatter data collected under simultaneous exposure of a plurality of radiation sources. In other words, the scatter correction method in this embodiment can be applicable to a single-source or a multi-source exposure mode.

Herein, the collection of the scatter image by the scatter detector and the preprocessing of the scatter image are existing technologies, and therefore details are not described. In an ideal condition, the preprocessing may not be performed.

Scatter data is extracted into an array based on preprocessed data, and the scatter data is caused to correspond to the bed position and the angle, where the array includes data information of each bed position and each angle, and specifically includes the following steps.

S30: Extract a scatter value corresponding to each pixel, and a bed position and an angle corresponding to each pixel.

A bed body control unit in a CT scan obtains current bed position information. The bed position and the angle are the most basic information for CT scanning. The bed position and the angle of each data collection are stored every time an image is captured. The bed position is a position of a bed, and the angle is angle information.

An object under test is a water phantom or a body phantom, which is an existing technology, and details are not described herein.

The scatter value detected by each pixel in the scatter detector is stored in association with the bed position and the angle.

S40: Obtain, within a bed position and angle plane, a scatter value of each grid position within the plane through interpolation based on a set bed position and angle grid.

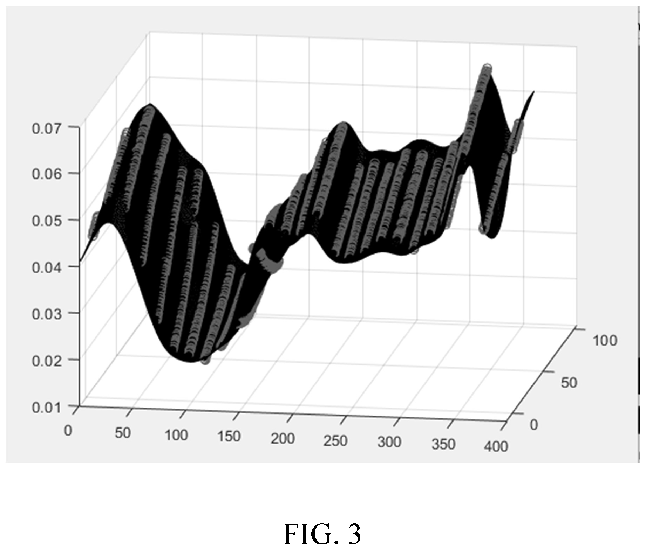

Referring to FIG. 3, x coordinates represent an angle; y coordinates represent a bed position; z coordinates represent a scatter value; and gray circular points represent measured scatter values, and the entire gray curved surface is obtained by interpolation based on the measured values.

The manner of interpolation includes at least cube interpolation or biharmonic spline interpolation, and compute unified device architecture (CUDA) acceleration is performed during the interpolation.

S50: Arrange, based on a detection direction of the main detector, the scatter values obtained through interpolation, and perform smoothing processing.

The detection direction of the main detector is an x direction of a main detector array. In this case, only the scatter value obtained through interpolation is used, and a scatter value obtained through detection is not considered. If the coordinates (x, y) exactly coincide, the scatter value obtained through interpolation is exactly the same as the scatter value obtained through detection.

S60: Store scatter distributions and corresponding bed positions and the angles of all the main detectors in the detection direction, to form a scatter correction table.

Specifically, scatter distributions of a plurality of main detectors are added and stored, to form the scatter correction table.

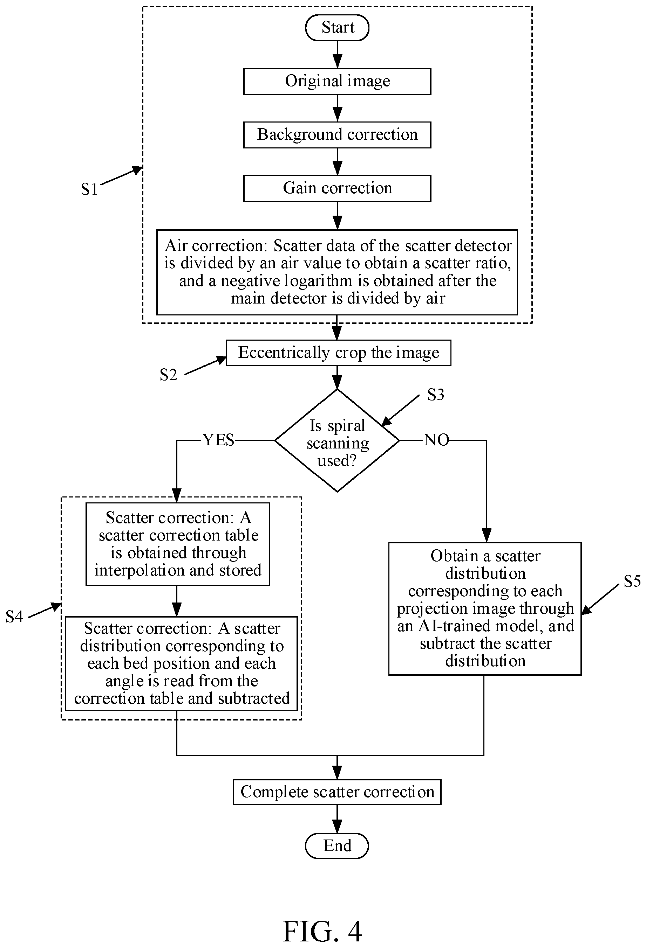

On this basis, FIG. 4 is a flowchart of a scatter correction method for static CT according to an embodiment of the present invention. In actual use, a patient is on a bed body, and a CT scan is started to obtain an original image. Then the method provided in this embodiment of the present invention is used to perform scatter correction, specifically including steps S1 to S5.

S1: Pre-correct the original image to obtain a first image.

Specifically, steps S11 to S13 are included.

S11: Perform background correction on the original image, to obtain a background-corrected image.

S12: Perform gain correction on the background-corrected image, to obtain a gain-corrected image.

S13: Perform air correction on the gain-corrected image, to obtain the first image.

Air data is a set of data obtained by performing a series of scans within a scanning range with no object placed. The actual scanning data is then divided by reference values obtained from air-only scanning, and a negative logarithm (-log) of a result of the division is calculated. Specifically, in an embodiment of the present invention, a process of air correction includes the following steps. The scatter data obtained by scatter detectors is divided by air values to obtain a scatter ratio. The air values are divided by the air values through the main detectors, to perform a negative logarithm operation and obtain the first image. In addition, background correction and gain correction are both common general knowledge in the art. Details are not described herein.

It should be noted that, unless otherwise specified, the original image, the first image, a second image, and the like in the present invention all refer to images detected by using the main detector.

S2: Eccentrically crop the first image to obtain a second image.

In this embodiment, the static CT is a full-ring detector, and the detector irradiated by each radiation source is fixed. Therefore, a range of image cutting is determined based on eccentricity data obtained through correction, to obtain the projection image corresponding to the source, that is, the second image. This is an existing technology, and therefore details are not described.

S3: Determine whether a CT scanning mode of the user is helical scanning.

Specifically, based on parameters collected during CT scanning based on static CT, it is determined by reading a parameter configuration whether the CT scanning mode is helical scanning. A process of determining, through the system parameter configuration, whether the CT scanning mode is helical scanning is common knowledge in this field. Details are not described herein.

S4: Perform scatter correction on the second image based on the scatter correction table if a determining result is yes, where the scatter correction table includes a scatter distribution corresponding to each bed position and each angle.

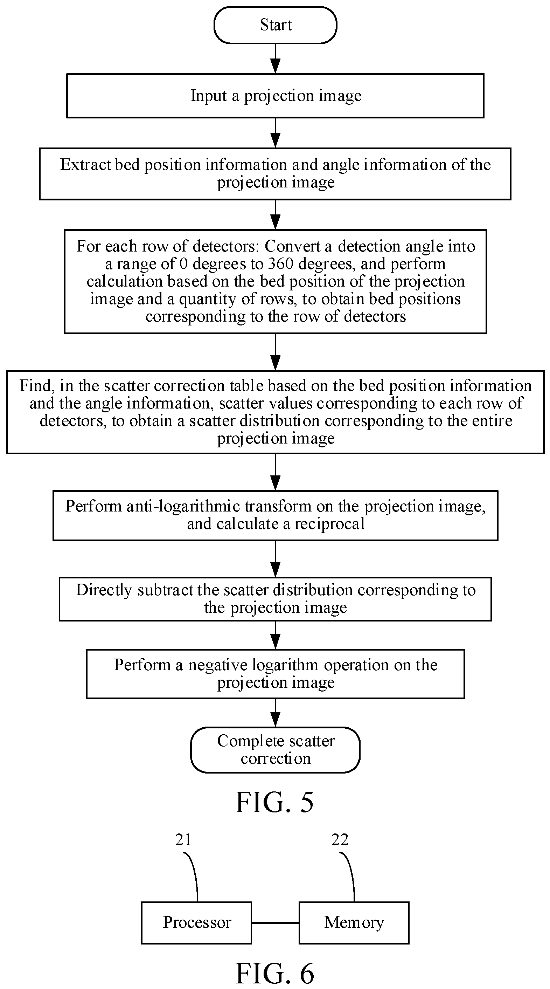

Referring to FIG. 5, the step specifically includes steps S41 to S47.

S41: Input a second image (that is, a projection image, the same below).

S42: Extract bed position information and angle information corresponding to the second image.

S43: Convert a detection angle into a range of 0 degrees to 360 degrees for each row of main detectors, and perform calculation based on the bed position and a quantity of rows of the second image, to obtain bed positions corresponding to the row of main detectors.

For example, an angle of a specific captured projection image is 10°, and a bed position is 20 mm. A bed position corresponding to each row of detectors in a Z direction of the main detectors is 0.165 mm. Accordingly, a bed position of the 30^{th} row of detectors is 20 mm + 30*0.165 mm, and the angle remains 10°.

It may be understood that in a static CT scanning system, the main detector is a detector ring assembled by a plurality of main detector modules. A row of main detectors herein refers to a plurality of main detector modules arranged along the Z direction.

A specific calculation process is as follows.

Assuming that the bed position corresponding to the second image is a, a width of the main detector in the z direction is b, a magnification factor from a rotation center to the main detector is c, and the number of a row in which the main detector is located is d, a calculation result of the bed position corresponding to the row of main detectors is: a+b*d/c.

S44: Find, in the scatter correction table based on the bed position and the angle information corresponding to the row of detectors, scatter values corresponding to each row of detectors, to obtain the scatter distribution corresponding to the second image.

S45: Perform anti-logarithmic transform on the second image, and perform reciprocal calculation, to obtain an image without being subjected to scatter correction.

S46: Subtract, from the obtained image without being subjected to the scatter correction, the scatter distribution corresponding to the second image, to obtain a scatter-corrected image.

S47: Perform a negative logarithm operation on the scatter-corrected image, to complete the scatter correction on the second image.

S5: Obtain a scatter distribution corresponding to the second image based on the pre-trained model, and perform scatter correction, if a determining result is no.

Specifically, in this embodiment, an operating principle of the pre-trained model is as follows:
obtaining the scatter data collected by the scatter detector, and projection data corresponding to the second image;
inputting the scatter data and the projection image data together into the pre-trained model; and
outputting, based on the pre-trained model, the scatter distribution corresponding to the second image.

It may be understood that the foregoing steps S4 and S5 are parallel steps and have no fixed sequence. Different data processing processes are performed based on different CT scanning modes to complete the scatter correction.

In addition, in the foregoing embodiment, when a CT scanning device is in an axial scanning mode, if both sides of the main detector are provided with scatter detectors, a scatter value of each pixel on the main detector is obtained through planar interpolation by using scatter values measured by the scatter detectors on both sides and a low-frequency characteristic of the scatter distribution, to perform scatter subtraction. If only one side of the main detector is provided with the scatter detector, a scatter value of each pixel on the main detector is estimated by using a scatter kernel convolution method or an AI method, to perform scatter subtraction.

Moreover, preferably, an arrangement spacing of the scatter detectors is greater than an arrangement spacing of the main detectors; and a larger arrangement spacing leads to a sparser arrangement, and a smaller arrangement spacing leads to a denser arrangement. Accordingly, the low-frequency characteristic of the spatial distribution of scatter intensity can be utilized to arrange the scatter detectors sparsely. This enables a more flexible layout of the scatter detectors. Neither blocking by the main detectors nor adoption of detectors with the same pixel size and density as the main detectors is required, which further saves costs.

Based on the foregoing scatter correction method for static CT, the present invention further provides a scatter correction system for static CT. As shown in FIG. 6, the scatter correction system includes one or more processors 21 and a memory 22. The memory 22 is coupled to the processor 21 and is configured to store a program. When the program is executed by the processor 21, the processor 21 is enabled to implement the scatter correction method for static CT in the foregoing embodiment.

The processor 21 is configured to control an overall operation of the scatter correction system, to complete all or part of the steps of the foregoing scatter correction method for static CT. The processor 21 may be a central processing unit (CPU), a graphics processing unit (GPU), a field-programmable gate array (FPGA), an application-specific integrated circuit (ASIC), a digital signal processing (DSP) chip, or the like. The memory 22 is configured to store various types of data to support operations in the scatter correction system. The data may include, for example, instructions of any application program or method for operation in the scatter correction system, and data related to the application program. The memory 22 may be implemented by any type of volatile or non-volatile storage device or a combination thereof, for example, a static random access memory (SRAM), an electrically erasable programmable read-only memory (EEPROM), an erasable programmable read-only memory (EPROM), a programmable read-only memory (PROM), a read-only memory (ROM), a magnetic memory, and a flash memory.

In an exemplary embodiment, the scatter correction system may be specifically implemented by a computer chip or an entity, or implemented by a product having a certain function, and is configured to perform the foregoing scatter correction method for static CT, and achieve the same technical effect as the foregoing method. A typical embodiment is a computer. Specifically, the computer may be, for example, a personal computer, a laptop computer, an on-board human-computer interaction device, a cellular phone, a camera phone, a smartphone, a personal digital assistant, a media player, a navigation device, an email device, a game console, a tablet computer, a wearable device, or any combination of these devices.

In another exemplary embodiment, the present invention further provides a computer-readable storage medium including program instructions. The program instructions, when executed by a processor, implement the steps of the scatter correction method for static CT in any one of the foregoing embodiments. For example, the computer-readable storage medium may be the foregoing memory including the program instructions, and the foregoing program instructions may be executed by the processor of the scatter correction system to complete the foregoing scatter correction method for static CT, and achieve the same technical effect as the foregoing method.

Based on the above, the scatter correction method for static CT and the scatter correction system for static CT provided in embodiments of the present invention have the following beneficial effects:
1. Scatter detectors are used to measure scatter in real time during CT scanning. By taking advantage of the characteristics of static CT, a scatter distribution at all positions and angles of a scanned object can be accurately obtained through interpolation, to perform precise scatter correction, thereby improving the effect of scatter correction.
2. The scatter correction method is easy to implement, and has low algorithm complexity.
3. The method may be widely applied to single-source and multi-source exposure modes.

The scatter correction method for static CT and the scatter correction system for static CT provided in the present invention are described in detail above. For a person of ordinary skill in the art, any apparent change made to the present invention without departing from the essential content of the present invention constitutes infringement of the patent right of the present invention and bears a corresponding legal responsibility.

## Claims

1. A scatter correction method for static CT, comprising the following steps:
collecting, by a scatter detector arranged on at least one side of a main detector, scatter data in advance, to obtain a scatter correction table through interpolation, and performing model training through the scatter data, to obtain a pre-trained model, wherein the scatter correction table comprises a scatter distribution corresponding to each bed position and each angle;
obtaining an original image of a user for performing a static CT scan;
pre-correcting the original image to obtain a first image;
eccentrically cropping the first image to obtain a second image;
determining whether a CT scanning mode of the user is helical scanning;
performing scatter correction on the second image by using the scatter correction table if a determining result is yes; and obtaining a scatter distribution corresponding to the second image based on the pre-trained model, and performing scatter correction on the second image, if a determining result is no.

2. The scatter correction method according to claim 1, wherein the scatter correction table is obtained in the following manner:
collecting a scatter image based on the scatter detector arranged on the at least one side of the main detector, and performing data preprocessing;
extracting, into an array, scatter data based on preprocessed data, and causing the scatter data to correspond to the bed position and the angle, wherein the array comprises data information of each bed position and each angle;
extracting, based on the scatter data, all scatter values of each pixel, and a bed position and an angle corresponding to each pixel;
obtaining, within a bed position and angle plane, a scatter value of each grid position within the plane through interpolation based on a set bed position and angle grid;
arranging, based on a detection direction of the main detector, the scatter values obtained through interpolation, and performing smoothing processing; and
storing scatter distributions and corresponding bed positions and the angles of all the main detectors in the detection direction, to form the scatter correction table.

3. The scatter correction method according to claim 2, wherein the performing scatter correction based on the scatter correction table specifically comprises:
inputting the second image;
extracting bed position information and angle information of the second image;
converting a detection angle into a range of 0 degrees to 360 degrees for each row of main detectors, and performing calculation based on the bed position and a quantity of rows of the second image, to obtain bed positions corresponding to the row of main detectors;
finding, in the scatter correction table based on the bed position and the angle information corresponding to the row of detectors, scatter values corresponding to each row of detectors, to obtain the scatter distribution corresponding to the second image;
performing anti-logarithmic transform on the second image, and performing reciprocal calculation, to obtain an image without being subjected to scatter correction;
subtracting, from the obtained image without being subjected to the scatter correction, the scatter distribution corresponding to the second image, to obtain a scatter-corrected image; and
performing a negative logarithm operation on the scatter-corrected image, to complete the scatter correction on the second image.

4. The scatter correction method according to claim 1, wherein the obtaining a scatter distribution corresponding to the second image based on the pre-trained model specifically comprises:
obtaining the scatter data collected by the scatter detector, and projection data corresponding to the second image;
inputting the scatter data and the projection image data together into the pre-trained model; and
outputting, based on the pre-trained model, the scatter distribution corresponding to the second image.

5. The scatter correction method according to claim 2, wherein
the manner of interpolation comprises at least cube interpolation or biharmonic spline interpolation, and compute unified device architecture (CUDA) acceleration is performed during the interpolation.

6. The scatter correction method according to claim 1, wherein the pre-correcting the original image specifically comprises:
performing background correction on the original image, to obtain a background-corrected image;
performing gain correction on the background-corrected image, to obtain a gain-corrected image; and
performing air correction on the gain-corrected image, to obtain the first image.

7. The scatter correction method according to claim 1, wherein
in an axial scanning mode, if both sides of the main detector are provided with scatter detectors, a scatter value of each pixel on the main detector is obtained through planar interpolation by using scatter values measured by the scatter detectors on both sides and a low-frequency characteristic of the scatter distribution, to perform scatter subtraction; or
if only one side of the main detector is provided with the scatter detector, a scatter value of each pixel on the main detector is estimated by using a scatter kernel convolution method or an AI method, to perform scatter subtraction.

8. The scatter correction method according to claim 1, wherein
an arrangement spacing of the scatter detectors is greater than an arrangement spacing of the main detectors; and a larger arrangement spacing leads to a sparser arrangement, and a smaller arrangement spacing leads to a denser arrangement.

9. The scatter correction method according to claim 1, wherein the collecting, by a scatter detector, scatter data comprises at least:
scatter data collected under exposure of a single radiation source; or scatter data collected under alternating exposure of a plurality of radiation sources; or scatter data collected under simultaneous exposure of a plurality of radiation sources.

10. A scatter correction system for static CT, comprising a processor and a memory, wherein the processor reads a computer program in the memory, and is configured to perform the following operations:
collecting, by a scatter detector arranged on at least one side of a main detector, scatter data in advance, to obtain a scatter correction table through interpolation, and performing model training through the scatter data, to obtain a pre-trained model, wherein the scatter correction table comprises a scatter distribution corresponding to each bed position and each angle;
obtaining an original image of a user for performing a static CT scan;
pre-correcting the original image to obtain a first image;
eccentrically cropping the first image to obtain a second image;
determining whether a CT scanning mode of the user is helical scanning;
performing scatter correction on the second image by using the scatter correction table if a determining result is yes; and obtaining a scatter distribution corresponding to the second image based on the pre-trained model, and performing scatter correction on the second image, if a determining result is no.
